# EUROPEAN PATENT SPECIFICATION

(11) **EP 2 021 006 B1**
(45) Date of publication and mention of the grant of the patent: **12.08.2015**
(21) Application number: 07728833.0
(22) Date of filing: 07.05.2007
(51) Int. Cl.: A61K 31/496, A61P 15/12, A61P 15/00

(54) **USE OF FLIBANSERIN FOR THE TREATMENT OF POST-MENOPAUSAL SEXUAL DESIRE DISORDERS**
VERWENDUNG VON FLIBANSERIN ZUR BEHANDLUNG VON POSTMENOPAUSALEN STÖRUNGEN DES SEXUELLEN VERLANGENS
UTILISATION DE FLIBANSERINE POUR LE TRAITEMENT DES TROUBLES DE LA LIBIDO POST-MENOPAUSIQUES

(30) Priority: 09.05.2006 US 746817 P; 14.07.2006 US 830987 P
(43) Date of publication of application: 11.02.2009
(73) Proprietor: Sprout Pharmaceuticals, Inc., Raleigh, NC 27609 (US)
(72) Inventor: POLLENTIER, Stephane, 1871 AW Schoorl (NL); PYKE, Robert, P.O.Box 368 Ridgefield, CT 06877 (US)
(74) Representative: Hally, Anna-Louise
(86) International application number: PCT/EP2007/054380
(87) International publication number: WO 2007/128802

(56) References cited:
- WO-A-03/035072
- WO-A-2005/007166
- WO-A-2005/102343
- FOURCROY J L: "Female sexual dysfunction: Potential for pharmacotherapy" DRUGS, ADIS INTERNATIONAL LTD, AT, vol. 63, no. 14, 2003, pages 1445-1457, XP009050771 ISSN: 0012-6667

## Description

The invention relates to the use of flibanserin for the preparation of a medicament for the treatment of post-menopausal Sexual Desire Disorders.

### Description of the invention

The compound 1-[2-(4-(3-trifluoromethyl-phenyl)piperazin-1-yl)ethyl]-2,3-dihydro-1H-benzimidazol-2-one (flibanserin) is disclosed in form of its hydrochloride in European Patent Application EP-A-526434 and has the following chemical structure:

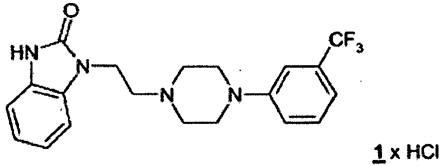

Flibanserin shows affinity for the 5-HT_{1A} and 5-HT₂-receptor. It is therefore a promising therapeutic agent for the treatment of a variety of diseases, for instance depression, schizophrenia, and anxiety.

The generic term "Sexual Disorders" includes Sexual Desire Disorders, Sexual Arousal Disorders, Orgasmic Disorders, Sexual Pain Disorders, Sexual Dysfunction due to a General Medical Condition, Substance-Induced Sexual Dysfunction, and Sexual Dysfunction not otherwise specified (Diagnostic and Statistical Manual of Mental Disorders, 4th edition, Text Revision. Washington DC, American Psychiatric Association, 2000).

The instant invention relates to the use of flibanserin, optionally in form of the free base, the pharmacologically acceptable acid addition salts and/or optionally in form of the hydrates and/or solvates thereof for the preparation of a medicament for the treatment of acquired hypoactive Sexual Desire Disorders in post-menopausal women, characterised in that 100mg flibanserin is administered once daily in the evening only consecutively over time.

Within the present invention the terms "treatment of post-menopausal Hypoactive Sexual Desire Disorder" etc. have the meaning of "treatment of Hypoactive Sexual Desire Disorders in post-menopausal women" etc.

The beneficial effects of flibanserin can be observed regardless of whether the Sexual Desire Disorder existed lifelong or was acquired, is of the "generalized type" or "situational type" and independent of etiologic origin (organic - both, physically and drug induced-, psychogen (due to psychological factors), a combination of organic - both, physically and drug induced-, and psychogen (due to psychological factors), or unknown). The term "lifelong" refers to such Sexual Desire Disorders of the present invention, which have been present since the onset of sexual functioning. The term "acquired" refers to such Sexual Desire Disorders of the present invention which developed only after a period of normal sexual functioning. The "generalized type" refers to such Sexual Disorders of the present invention wherein the disorder is not limited to certain types of stimulation, situations, or partners. The "situational type" applies to such Sexual Disorders of the present invention wherein the disorder is limited to certain types of stimulation, situations, or partners. The subtype due to "psychological factors" applies when psychological factors are judged to have the major role in the onset, severity, exacerbation, or maintenance of the Sexual Disorder, and general medical conditions and substance play no role in the etiology of the Sexual Disorder. Finally the subtype due to "combined factors" applies when 1) psychological factors are judged to have a role in the onset, severity, exacerbation, or maintenance of the Sexual Disorder, and 2) a general medical condition or substance use is also judged to be contributory but is not sufficient to account for a Sexual Disorder (Diagnostic and Statistical Manual of Mental Disorders, 4th edition, Text Revision. Washington DC, American Psychiatric Association, 2000).

Therefore, e.g. the term "lifelong post-menopausal Hypoactive Sexual Desire Disorder" refers to Hypoactive Sexual Desire Disorder in post-menopausal women which has been present since the onset of sexual functioning and the term "acquired post-menopausal Hypoactive Sexual Desire Disorder" refers to Hypoactive Sexual Desire Disorder in post-menopausal women, which developed after a period of normal sexual functioning. Although there may seem to be an apparent contradiction in the wording "lifelong post-menopausal" this should be understood as a disorder diagnosed after the menopause whereby history reveals that the disorder in fact was present since the onset of sexual functioning.

Furthermore the present invention relates to the generalized or situational subtype of any of the above mentioned conditions and/or to such which are due to psychological factors or due to combined factors.

Flibanserin can optionally used in form of the free base, in form of its pharmaceutically acceptable acid addition salts and/or optionally in form of the hydrates and/or solvates thereof. Suitable acid addition salts include for example those of the acids selected from, succinic acid, hydrobromic acid, acetic acid, fumaric acid, maleic acid, methanesulphonic acid, lactic acid, phosphoric acid, hydrochloric acid, sulphuric acid, tartaric acid and citric acid. Mixtures of the abovementioned acid addition salts may also be used. From the aforementioned acid addition salts the hydrochloride and the hydrobromide, particularily the hydrochloride, are preferred. If flibanserin is used in form of the free base, it is preferably used in form of flibanserin polymorph A as disclosed in WO 03/014079.

Flibanserin, optionally used in form of the free base, the pharmacologically acceptable acid addition salts and/or optionally in form of the hydrates and/or solvates thereof, may be incorporated into the conventional pharmaceutical preparation in solid, liquid or spray form. The composition may, for example, be presented in a form suitable for oral, rectal, parenteral administration or for nasal inhalation: preferred forms includes for example, capsules, tablets, coated tablets, ampoules, suppositories and nasal spray.

The active ingredient may be incorporated in excipients or carriers conventionally used in pharmaceutical compositions such as, for example, talc, arabic gum, lactose, gelatine, magnesium stearate, corn starch, acqueous or non acqueous vehicles, polyvynil pyrrolidone, semisynthetic glicerides of fatty acids, benzalconium chloride, sodium phosphate, EDTA, polysorbate 80. The compositions are advantageously formulated in dosage units, each dosage unit being adapted to supply a single dose of the active ingredient.

The compounds of the invention are administered once daily consecutively over a period of time.

The dose is administered to a patient once in the evening only (50 or 100 mg of flibanserin) consecutively over a period of time. In order to improve tolerability for a short period half the target dose can be administered.

As a result side-effects such as sedation are of lesser significance.

Suitable tablets may be obtained, for example, by mixing the active substance(s) with known excipients, for example inert diluents such as calcium carbonate, calcium phosphate or lactose, disintegrants such as corn starch or alginic acid, binders such as starch or gelatine, lubricants such as magnesium stearate or talc and/or agents for delaying release, such as carboxymethyl cellulose, cellulose acetate phthalate, or polyvinyl acetate. The tablets may also comprise several layers.

Coated tablets may be prepared accordingly by coating cores produced analogously to the tablets with substances normally used for tablet coatings, for example collidone or shellac, gum arabic, talc, titanium dioxide or sugar. To achieve delayed release or prevent incompatibilities the core may also consist of a number of layers. Similarly the tablet coating may consist of a number or layers to achieve delayed release, possibly using the excipients mentioned above for the tablets.

Syrups or elixirs containing the active substances or combinations thereof according to the invention may additionally contain a sweetener such as saccharine, cyclamate, glycerol or sugar and a flavour enhancer, e.g of a flavouring such as vanilline or orange extract. They may also contain suspension adjuvants or thickeners such as sodium carboxymethyl cellulose, wetting agents such as, for example, condensation products of fatty alcohols with ethylene oxide, or preservatives such as p-hydroxybenzoates.

Solutions for injection are prepared in the usual way, e.g of. with the addition of preservatives such as p-hydroxybenzoates, or stabilisers such as alkali metal salts of ethylenediamine tetraacetic acid, and transferred into injection vials or ampoules.

Capsules containing one or more active substances or combinations of active substances may for example be prepared by mixing the active substances with inert carriers such as lactose or sorbitol and packing them into gelatine capsules.

Suitable suppositories may be made for example by mixing with carriers provided for this purpose, such as neutral fats or polyethyleneglycol or the derivatives thereof.

The Examples which follow illustrate the present invention without restricting its scope:

### Examples and comparative examples of pharmaceutical formulations

| A) | Tablets | per tablet |
|---|---|---|
| | flibanserin | 100 mg |
| | lactose | 240 mg |
| | corn starch | 340 mg |
| | polyvinylpyrrolidone | 45 mg |
| | magnesium stearate | 15 mg |
| | | 740 mg |

The finely ground active substance, lactose and some of the corn starch are mixed together. The mixture is screened, then moistened with a solution of polyvinylpyrrolidone in water, kneaded, wet-granulated and dried. The granules, the remaining corn starch and the magnesium stearate are screened and mixed together. The mixture is compressed to produce tablets of suitable shape and size.

| B) | Tablets | per tablet |
|---|---|---|
| | flibanserin | 80 mg |
| | corn starch | 190 mg |
| | lactose | 55 mg |
| | microcrystalline cellulose | 35 mg |
| | polyvinylpyrrolidone | 15 mg |
| | sodium-carboxymethyl starch | 23 mg |
| | magnesium stearate | 2 mg |
| | | 400 mg |

The finely ground active substance, some of the corn starch, lactose, microcrystalline cellulose and polyvinylpyrrolidone are mixed together, the mixture is screened and worked with the remaining corn starch and water to form a granulate which is dried and screened. The sodium-carboxymethyl starch and the magnesium stearate are added and mixed in and the mixture is compressed to form tablets of a suitable size.

| C) | Coated tablets | per coated tablet |
|---|---|---|
| | flibanserin | 5 mg |
| | corn starch | 41.5 mg |
| | lactose | 30 mg |
| | polyvinylpyrrolidone | 3 mg |
| | magnesium stearate | 0.5 mg |
| | | 80 mg |

The active substance, corn starch, lactose and polyvinylpyrrolidone are thoroughly mixed and moistened with water. The moist mass is pushed through a screen with a 1 mm mesh size, dried at about 45°C and the granules are then passed through the same screen. After the magnesium stearate has been mixed in, convex tablet cores with a diameter of 6 mm are compressed in a tablet-making machine. The tablet cores thus produced are coated in known manner with a covering consisting essentially of sugar and talc. The finished coated tablets are polished with wax.

| D) | Capsules | per capsule |
|---|---|---|
| | flibanserin | 1 50 mg |
| | Corn starch | 268.5 mg |
| | Magnesium stearate | 1.5 mg |
| | | 420 mg |

The substance and corn starch are mixed and moistened with water. The moist mass is screened and dried. The dry granules are screened and mixed with magnesium stearate. The finished mixture is packed into size 1 hard gelatine capsules.

| E) | Ampoule solution | |
|---|---|---|
| | flibanserin | 50 mg |
| | sodium chloride | 50 mg |
| | water for inj. | 5 ml |

The active substance is dissolved in water at its own pH or optionally at pH 5.5 to 6.5 and sodium chloride is added to make it isotonic. The solution obtained is filtered free from pyrogens and the filtrate is transferred under aseptic conditions into ampoules which are then sterilised and sealed by fusion.

| F) | Suppositories | |
|---|---|---|
| | flibanserin | 50 mg |
| | solid fat | 1650 mg |
| | | 1700 mg |

The hard fat is melted. At 40°C the ground active substance is homogeneously dispersed. It is cooled to 38°C and poured into slightly chilled suppository moulds.

## Claims

1. Use of flibanserin, optionally in form of the free base, the pharmacologically acceptable acid addition salts and/or optionally in form of the hydrates and/or solvates thereof for the preparation of a medicament for the treatment of acquired Hypoactive Sexual Desire Disorder in postmenopausal women, **characterized in that** 100mg flibanserin is administered once daily in the evening only consecutively over a period of time.

2. Flibanserin, optionally in form of the free base, the pharmacologically acceptable acid addition salts and/or optionally in form of the hydrates and/or solvates thereof, for use in the treatment, by once daily administration, of acquired Hypoactive Sexual Desire Disorders in postmenopausal women **characterized in that** 100mg flibanserin is administered once daily in the evening only consecutively over a period of time.

3. Use or flibanserin for use in the treatment according to one or more of the preceding claims, **characterized in that** the Sexual Desire Disorder is of the generalized subtype.

4. Use or flibanserin for use in the treatment according to one or more of the preceding claims, **characterized in that** the Sexual Desire Disorder is of the situational subtype.

5. Use or flibanserin for use in the treatment according to one or more of the preceding claims, **characterized in that** the Sexual Desire Disorder is due to psychological factors.

6. Use or flibanserin for use in the treatment according to one or more of the preceding claims, **characterized in that** the Sexual Desire Disorder is due to combined factors.

7. Use or flibanserin for use in the treatment according to one or more of the preceding claims, **characterized in that** flibanserin is applied in form of a pharmaceutically acceptable acid addition salt selected from the salts formed by the acids selected from, succinic acid, hydrobromic acid, acetic acid, fumaric acid, maleic acid, methanesulphonic acid, lactic acid, phosphoric acid, hydrochloric acid, sulphuric acid, tartaric acid, citric acid, and mixtures thereof.

8. Use or flibanserin for use in the treatment according to one or more of the preceding claims, **characterized in that** flibanserin is applied in form of its free base.

9. Use or flibanserin for use in the treatment according to claim 8, **characterized in that** flibanserin is applied in form of a polymorph A of the free base, having a melting point of about 161°C as measured using DSC.

## Patentansprüche

1. Verwendung von Flibanserin, optional in der Form der freien Base, der pharmakologisch verträglichen Säureadditionssalze und/oder optional in der Form der Hydrate und/oder Solvate davon, für die Herstellung eines Medikaments für die Behandlung von erworbenem vermindertem sexuellem Verlangen (Hypoactive Sexual Desire Disorder, HSDD) bei postmenopausalen Frauen, **dadurch gekennzeichnet, dass** 100 mg Flibanserin einmal täglich nur am Abend aufeinanderfolgend über einen Zeitraum verabreicht wird.

2. Flibanserin, optional in der Form der freien Base, der pharmakologisch verträglichen Säureadditionssalze und/oder optional in der Form der Hydrate und/oder Solvate davon, für die Verwendung bei der Behandlung, durch einmal tägliche Verabreichung, von erworbenem vermindertem sexuellem Verlangen bei postmenopausalen Frauen, **dadurch gekennzeichnet, dass** 100 mg Flibanserin einmal täglich nur am Abend aufeinanderfolgend über einen Zeitraum verabreicht wird.

3. Verwendung oder Flibanserin für die Verwendung bei der Behandlung nach einem oder mehreren der vorstehenden Ansprüche, **dadurch gekennzeichnet, dass** die Störung des sexuallen Verlangens den allgemeinen Subtyp darstellt.

4. Verwendung oder Flibanserin für die Verwendung bei der Behandlung nach einem oder mehreren der vorstehenden Ansprüche, **dadurch gekennzeichnet, dass** die Störung des sexuallen Verlangens den situationsbedingten Subtyp darstellt.

5. Verwendung oder Flibanserin für die Verwendung bei der Behandlung nach einem oder mehreren der vorstehenden Ansprüche, **dadurch gekennzeichnet, dass** die Störung des sexuallen Verlangens aufgrund von psychologischen Faktoren vorliegt.

6. Verwendung oder Flibanserin für die Verwendung bei der Behandlung nach einem oder mehreren der vorstehenden Ansprüche, **dadurch gekennzeichnet, dass** die Störung des sexuallen Verlangens aufgrund von kombinierten Faktoren vorliegt.

7. Verwendung oder Flibanserin für die Verwendung bei der Behandlung nach einem oder mehreren der vorstehenden Ansprüche, **dadurch gekennzeichnet, dass** Flibanserin in Form eines pharmazeutisch verträglichen Säureadditionssalzes verabreicht wird, das aus den Salzen ausgewählt ist, die durch die Säuren gebildet werden, die aus Folgenden ausgewählt sind: Bernsteinsäure, Bromwasserstoffsäure, Essigsäure, Fumarsäure, Maleinsäure, Methansulfonsäure, Milchsäure, Phosphorsäure, Chlorwasserstoffsäure, Schwefelsäure, Weinsäure, Citronensäure und Mischungen davon.

8. Verwendung oder Flibanserin für die Verwendung bei der Behandlung nach einem oder mehreren der vorstehenden Ansprüche, **dadurch gekennzeichnet, dass** Flibanserin in Form seiner freien Base verabreicht wird.

9. Verwendung oder Flibanserin für die Verwendung bei der Behandlung nach Anspruch 8, **dadurch gekennzeichnet, dass** Flibanserin in Form eines Polymorphs A der freien Base verabreicht wird, das einen Schmelzpunkt von etwa 161°C aufweist, wie unter Verwendung von DSC gemessen wird.

## Revendications

1. Utilisation du flibanserin, en option sous la forme de la base libre, des sels d'addition d'acides pharmacologiquement acceptables et/ou, en option sous la forme des hydrates et/ou des solvates de celui-ci dans la préparation d'un médicament pour le traitement du trouble acquis du désir sexuel hypoactif chez des femmes post-ménopausées, **caractérisée en ce que** le flibanserin à 100 mg est administré une fois par jour en soirée consécutivement sur une certaine période de temps seulement.

2. Flibanserin, en option sous la forme de la base libre, des sels d'addition d'acides pharmacologiquement acceptables et/ou, en option sous la forme des hydrates et/ou des solvates de celui-ci dans le traitement, en administrations quotidiennes uniques, du trouble acquis du désir sexuel hypoactif chez des femmes post-ménopausées, **caractérisé en ce que** le flibanserin à 100 mg est administré une fois par jour en soirée consécutivement sur une certaine période de temps seulement.

3. Utilisation ou flibanserin pour un usage thérapeutique selon une ou plusieurs des revendications précédentes, **caractérisé en ce que** le trouble du désir sexuel est du sous-type généralisé.

4. Utilisation ou flibanserin pour un usage thérapeutique selon une ou plusieurs des revendications précédentes, **caractérisé en ce que** le trouble du désir sexuel est du sous-type situationnel.

5. Utilisation ou flibanserin pour un usage thérapeutique selon une ou plusieurs des revendications précédentes, **caractérisé en ce que** le trouble du désir sexuel est dû à des facteurs psychologiques.

6. Utilisation ou flibanserin pour un usage thérapeutique selon une ou plusieurs des revendications précédentes, **caractérisé en ce que** le trouble du désir sexuel est dû à des facteurs combinés.

7. Utilisation ou flibanserin pour un usage thérapeutique selon une ou plusieurs des revendications précédentes, **caractérisé en ce que** le flibanserin est appliqué sous la forme d'un sel d'addition d'acide pharmaceutiquement acceptable sélectionné parmi les sels formés par les acides sélectionnés parmi les suivants : acide succinique, acide bromhydrique, acide acétique, acide fumarique, acide maléique, acide méthanesulfonique, acide lactique, acide phosphorique, acide chlorhydrique, acide sulfurique, acide tartrique, acide nitrique et mélanges de ceux-ci.

8. Utilisation ou flibanserin pour un usage thérapeutique selon une ou plusieurs des revendications précédentes, **caractérisé en ce que** le flibanserin est appliqué sous la forme de sa base libre.

9. Utilisation ou flibanserin pour un usage thérapeutique selon la revendication 8, **caractérisé en ce que** le Flibanserin est appliqué sous la forme d'un polymorphe A de la base libre dont le point de fusion est d'environ 161 °C à la mesure par colorimétrie différentielle à balayage.
